(19) Europäisches Patentamt
European Patent Office
Office européen des brevets

(11) Veröffentlichungsnummer : **0 290 378 B1**

# (12) EUROPÄISCHE PATENTSCHRIFT

(45) Veröffentlichungstag der Patentschrift :
**02.01.92 Patentblatt 92/01**

(51) Int. Cl.$^5$ : **C07J 1/00**

(21) Anmeldenummer : **88730106.7**

(22) Anmeldetag : **06.05.88**

## (54) Verfahren zur Herstellung von 1-Methyl-androsta-1,4-dien-3,17-dion und die neuen Zwischenprodukte für dieses Verfahren.

(30) Priorität : **07.05.87 DE 3715869**

(43) Veröffentlichungstag der Anmeldung :
**09.11.88 Patentblatt 88/45**

(45) Bekanntmachung des Hinweises auf die Patenterteilung :
**02.01.92 Patentblatt 92/01**

(84) Benannte Vertragsstaaten :
**AT BE CH DE ES FR GB GR IT LI LU NL SE**

(56) Entgegenhaltungen :
**EP-A- 0 129 500**
**DE-A- 3 338 212**
**DE-A- 3 539 244**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **Nickisch, Klaus, Dr.**
**Alt-Lichtenrade 11**
**W-1000 Berlin 49 (DE)**
Erfinder : **Arnold, Hanfried, Dr.**
**Von-Wettstein-Strasse 4**
**W-1000 Berlin 33 (DE)**

EP 0 290 378 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von 1-Methyl-androsta-1,4-dien-3,17-dion (Metandroden) und die neuen Zwischenprodukte für dieses Verfahren. Metandroden ist ein Inhibitor der Östrogenbiosynthese (DOS 3322285).

Bisher wurde das 1-Methyl-androsta-1,4-dien-3,17-dion durch Oxidation von 17β-Hydroxy-1-methyl-androsta-1,4-dien-3-on (DOS 3322285) oder durch mikrobiologische Dehydrierung des 1-Methyl-5α-androst-1-en-3,17-dion (DOS 3512328) hergestellt. Die Herstellungen dieser beiden Verbindungen verlaufen selbst über mehrstufige Synthesen und in geringen Ausbeuten.

Ein noch nicht befriedigend gelöstes Problem ist die Einführung der $\Delta^1$-Doppelbindung am Steroid beim Vorhandensein einer 1α-Methylgruppe und einer $\Delta^4$-Doppelbindung.

Das 1-Methyl-androsta-1,4-dien-3,17-dion-System wird bisher aus dem entsprechenden 17β-Hydroxy-androsta-1,4-dien-3-on über die Synthesesequenz

a) kupferkatalysierte metallorganische Michael-Addition eines Methylanions (DE-AS 2046640),
b) Hydrierung der $\Delta^4$-Doppelbindung zur entsprechenden 5α-H-Verbindung,
c) Dibromierung zum 2,4-Dibromsteroid,
d) Zweifache Bromwasserstoffeliminierung und
e) Oxidation des 17-Alkohols zum 17-Keton

hergestellt (DE-OS 3338212 und DE-OS 3539244).

Bei diesem Syntheseweg wird die bereits vorhandene $\Delta^4$-Doppelbindung hydriert (Schutzgruppeneffekt), um dann anschließend zusammen mit der $\Delta^1$-Doppelbindung wieder eingeführt zu werden.

Dieses umständliche Verfahren muß angewendet werden, weil innerhalb des Standes der Technik kein geeignetes Verfahren bekannt ist, um aus einem 1α-Methyl-4-androsten-3,17-dion direkt zum 1-Methyl-androsta-1,4-dien-3,17-dion-System zu kommen.

Erschwert wird die Synthese nach der beschriebenen Synthesesequenz außerdem durch Reinigungsprobleme auf der Stufe der zweifachen Bromwasserstoffeliminierung. Das eingesetzte 2,4-Dibrom-17β-hydroxy-1α-methyl-5α-H-androstan-3-on reagiert unter den Reaktionsbedingungen außer zu dem gewünschten 17β-Hydroxy-1-methyl-androsta-1,4-dien-3-on noch zu dem unerwünschten 17β-Hydroxy-1α-methylandrosta-4,6-dien-3-on in gleicher Ausbeute [J.Am.Chem.Soc. 68, 1712 (1946) und Coll. Czech. Chem. Comm. 26, 1852 (1961)]. Dieses unerwünschte Nebenprodukt ist aufgrund ähnlicher Eigenschaften nur schwer abtrennbar (Chromatographie).

Es wurde nun gefunden, daß sich 1-Methyl-androsta-1,4-dien-3,17-dion über einen kürzeren Syntheseweg, ohne aufwendige chromatographische Reinigungsverfahren und in einer höheren Gesamtausbeute herstellen läßt. Für die Bromierung und Dehydrobromierung nach der Umwandlung des Androstan-1,4-dien-3,17-dions in das 3-Enoloxy-1α-methyl-androsta-2,4-dien-17-on wird ein einfacheres Verfahren entwickelt, bei dem gleichzeitig die beschriebenen Reinigungsprobleme auf der Stufe der zweifachen Bromwasserstoffeliminierung entfallen.

Ausgangsmaterial für die neue Synthese ist das leicht zugängliche Androsta-1,4-dien-3,17-dion [J. Am. Chem. Soc. 79, 3920 (1957), Tetrahedron 4, 201 (1958)].

Die Erfindung betrifft somit ein Verfahren zur Herstellung von 1-Methyl-androsta-1,4-dien-3,17-dion der Formel I

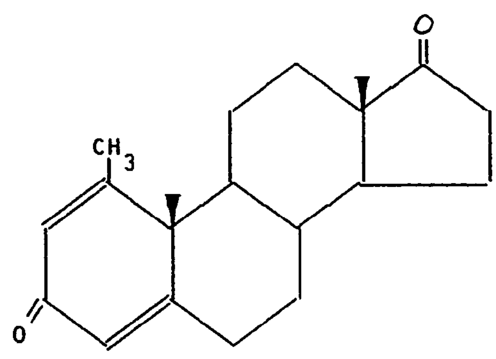

(I),

dadurch gekennzeichnet, daß man Androsta-1,4-dien-3,17-dion

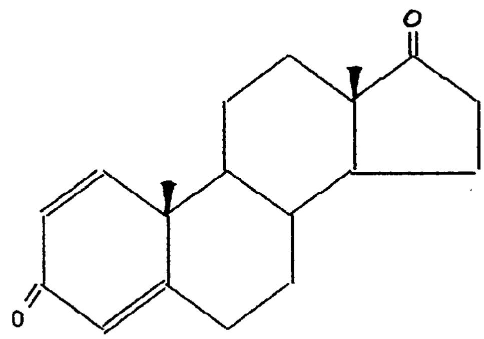

durch Umsetzung mit einem Methylanionen liefernden Reagenz und anschließendes Abfangen des gebildeten Enolats in einen 3-Enolester oder 3-Enolether der allgemeinen Formel II

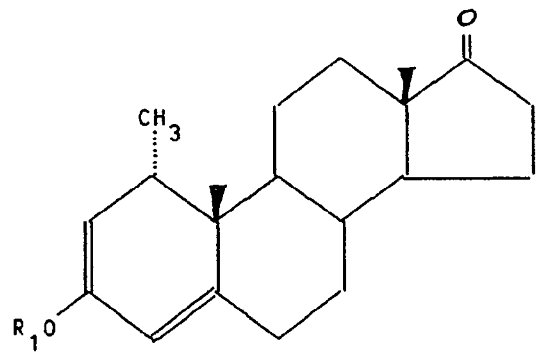

(II),

worin

$R_1$ einen Alkylrest mit 1-3 Kohlenstoffatomen oder eine Acyl- oder Trialkylsilyl gruppe mit bis zu 10 Kohlenstoffatomen in der Gruppe bedeutet,

überführt, diesen mit elementarem Brom oder einem Bromierungsreagenz, bei dem das aktive Brom an amidischen Stickstoff gebunden vorliegt, in das 2-Bromsteroid der Formel III

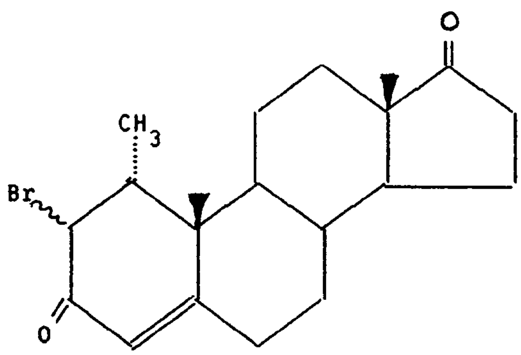

(III),

worin

der Bromsubstituent sowohl in der α- als auch in der β-Position stehen kann,

umsetzt, und die Verbindung der allgemeinen Formel III durch Dehydrobromierung in Gegenwart von Alkali- oder Erdalkylioxiden oder -carbonaten in die Verbindung der allgemeinen Formel I überführt.

Die Erfindung betrifft weiter Verbindungen der allgemeinen Formel II

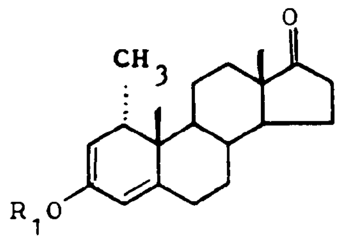

(II),

worin

$R_1$ einen Alkylrest mit 1-3 Kohlenstoffatomen oder eine Acyl- oder Trialkylsilylgruppe mit bis zu 10 Kohlenstoffatomen in der Gruppe bedeutet,

die als Zwischenprodukte des Verfahrens, durch Umsetzung von Androsta-1,4-dien-3,17-dion mit einem Methylanion im Sinne einer Michael-Addition und anschließendes Abfangen des Enolats, anfallen.

Als Rest $R_1$ der allgemeinen Formel II finden Alkylreste mit 1-3 Kohlenstoffatomen oder Acyl- oder Trialkylsilylreste mit bis zu 10 Kohlenstoffatomen in der Gruppe Anwendung. Besonders geeignet sind der Acetyl- oder Trimethylsilylrest.

Die Herstellung der Verbindung der allgemeinen Formel II erfolgt aus Androst-1,4-dien-3,17-dion durch Umsetzung mit einem Methylanion im Sinne einer Michael-Addition bei der das sich als Zwischenprodukt bildende Enol, als Enolderivat, abgefangen wird.

Das für die Michael-Addition (1,4-Addition) benötigte Methylanion wird wahlweise aus einem Grignard-Reagenz bei Anwesenheit von Kupfer-I-salzen, zum Beispiel Kupfer-I-jodid oder -bromid,oder aus einer Organokupferlithium-Verbindung, zum Beispiel Kupferdimthyllithium, gebildet.

Das Abfangen des erwähnten Enols erfolgt mit Orthoessigsäuretrimethylester, dem Triethylester, dem Tripropylester, oder auch mit 2,2-Dimethoxypropan, mit den Säureanhydriden oder Säurechloriden der organischen Carbonsäuren mit 1-10 Kohlenstoffatomen, mit Trimethylsilylchlorid, Tribenzylsilylchlorid, Dimethyl-tert.-butylsilylchlorid in inerten Lösungsmitteln, wie zum Beispiel Dichlormethan, Tetrachlorkohlenstoff, Dimethylsulfoxid oder Dimethylformamid.

Kupferdimethyllithium, welches in situ aus Kupfer(I)-jodid und Methyllithium in Dichlorethan hergestellt wird, reagiert am Steroid im Sinne einer 1,4-Addition. Das sich ausbildende Enolat wird vorzugsweise mit Acetanhydrid als Enolacetat abgefangen ; man erhält das 3-Acetoxy-1α-methyl-androsta-2,4-dien-17-on als kristallinen Stoff.

Dieses Derivat läßt sich nur über die beschriebenen Reaktionsschritte herstellen.

Bei der Umsetzung des entsprechenden 3-Keto-$\Delta^4$-Derivates unter den üblichen Herstellungsmethoden für Enolether und -acylate [J. Am. Chem. Soc. 82, 5488 (1960)] würde nicht das gewünschte 2,4-Dien-3-ol, sondern das $\Delta^{3,5}$-Dienol-Derivat [J. Org. Chem. 26, 976 (1961)] entstehen.

Die Einführung des Bromstoms in die 2-Stellung am Steroid wird durch Umsetzung einer Verbindung der allgemeinen Formel II zu einer Verbindung der allgemeinen Formel III durch Reaktion mit elementarem Brom oder mit einem Bromierungsreagenz, bei dem das aktive Brom an amidischen Stickstoff gebunden vorliegt, erreicht. Als Bromierungsreagenzien eignen sich zum Beispiel N-Bromsuccinimid oder 1,3-Dibrom-5,5-dimethylhydantoin.

Durch Bromierung mit 1,3-Dibrom-5,5-dimethyl-hydantoin in wäßrigem Dioxan erhält man aus dem 3-Acetoxy-1α-methyl-androsta-2,1-dien-17-on das 2β-Brom-1α-methyl-4-androsten-3,17-dion, welches mit wenig 2α-Brom-1α-methyl-4-androsten-3,17-dion verunreinigt ist.

Für die Bromierung eignen sich aber such andere polare aprotische Lösungsmittel, wie zum Beispiel Tetrahydrofuran, Aceton und Dichlormethan, oder Wasser. Geeignet ist such der Einsatz von Lösungsmittelgemischen, die sus zwei oder mehreren Komponenten bestehen, wie zum Beispiel die Mischungen Dioxan/Wasser (1 : 1) ; Dioxan/Aceton/Dichlormethan (3 : 1 : 2) ; und Tetrahydrofuran/Dioxan/Wasser (10 : 3 : 1).

Die Bromwasserstoffabspaltung zu der Verbindung der allgemeinen Formel I erfolgt aus der Bromverbindung durch Behandlung mit einem Alkili- oder Erdalkalioxid oder -carbonat in Dimethylformamid. Besonders gut vorläuft die Reaktion mit Magnesiumoxid. Die Zugabe der Bromverbindung erfolgt vorzugsweise in die vorgeheizte Reaktionsmischung in dem Maße, daß die Reaktionstemperatur von etwa 110°C nicht unterschritten wird. Es ist jedoch möglich, bei höheren Temperaturen als 110°C bis hin zur Siedetemperatur des Dimethylformamids zu arbeiten. Im Reaktionsgemisch werden neben dem gewünschten 1-Methyl-androsta-1,4-dien-3,17-dion nur ca. 5% 1α-Methylandrosta-1,6-dien-3,17-dion gefunden.

Als Lösungsmittel für die Dehydrobromierung eignen sich außer Dimethylformamid auch Dimethyacetamid, Dimethylimidazolidinon und Dimethylsulfoxid, allein, im Gemisch untereinander oder im Gemisch mit Wasser.

Auf die Einhaltung der optimierten Abspaltungsbedingungen muß wegen der bekannten Wanderungstendenz des 2-Bromsubstituenten in die 4- bzw. 6-Position, was Anlaß zur Bildung des $\Delta^{4,6}$-Steroids geben würde, geachtet werden [Coll. Czech. Chem. Comm. 26, 1852 (1961)].

**Beispiel 1**

3-Acetoxy-1α-methyl-androsta-2,4-dien-17-on

Eine Suspension von 15,38 g Kupfer-(I)-jodid in 50 ml Dichlorethan wird auf −30°C gekühlt, bei dieser Temperatur mit 100 ml Methyllithiumlösung versetzt und 1 Stunde nachgerührt. Dazu tropft man eine Lösung von 12,8 g Androsta-1,4-dien-3,17-dion [J. Am. Chem. Soc. 79, 3920 (1957) ; Tetrahedron 4, 201 (1958)] in 60 ml Dichlorethan so zu, daß die Temperatur −10°C nicht übersteigt. Nach erfolgter Zugabe wird 2 Stunden bei 0°C nachgerührt. Zu dieser gelb-braunen Lösung gibt man eine Lösung von 8,62 g Acetanhydrid in 22 ml Dichlorethan und rührt 1 Stunde bei Raumtemperatur nach. Anschließend tropft man eine Lösung von 8,3 g Ammoniumchlorid in 83 ml Wasser zu. Die organische Phase wird abgetrennt, die wäßrige Phase mit Methylenchlorid extrahiert und die vereinigten organischen Phasen mit Wasser gewaschen. Nach dem Trocknen über Natriumsulfat wird im Vakuum eingeengt und das erhaltene Rohprodukt aus Hexan/Aceton umkristallisiert. Man erhält 8,9 g 3-Acetoxy-1α-methyl-androsta-2,4-dien-17-on vom Schmelzpunkt 104-107°C.

**Beispiel 2**

2β-Brom-1α-methyl-4-androsten-3,17-dion und 2α-Brom-1α-methyl-4-androsten-3,17-dion

Eine Suspension von 5,18 g 3-Acetoxy-1α-methyl-androsta-2,4-dien-17-on in 50 ml Dioxan und 100 ml Wasser wird unter Eiskühlung portionsweise mit 2,2 g 1,3-Dibrom-5,5-dimethylhydantoin versetzt und 1 Stunde bei Raumtemperatur nachgerührt. Anschließend fällt man in 1 l mit Kochsalz gesättigtem Wasser, gibt 2,7 g Natriumsulfit zu und rührt 60 Minuten nach. Der ausgefallene Rückstand wird abfiltriert, mit Wasser chloridfrei gewaschen und getrocknet. Man erhält 5,65 g eines Gemisches von 2β-Brom-1α-methyl-4-androsten-3,17-dion und 2α-Brom-1α-methyl-4-androsten-3,17-dion.

**Beispiel 3**

1-Methyl-androsta-1,4-dien-3,17-dion

Zu einer auf 115 °C vorgeheizten Suspension von 1 g Magnesiumoxid in 30 ml Dimethylformamid werden 5 g eines Gemisches aus 2β-Brom- und 2α-Brom-1α-methyl-androst-4-en-3,17-dion so eingetragen, daß die Temperatur nicht unter 110°C fällt. Anschließend wird noch 2 Stunden bei dieser Temperatur nachgerührt, in 300 ml Eiwasser gefällt, filtriert und getrocknet. Das erhaltene Rohprodukte von 3,85 g wird anschließend aus Essigester/Hexan umkristallisiert. Man erhält 2,75 g 1-Methylandrosta-1,4-dien-3,17-dion vom Schmelzpunkt 165-167°C.

**Patentansprüche**

1. Verfahren zur Herstellung von 1-Methyl-androsta-1,4-dien-3,17-dion der Formel I

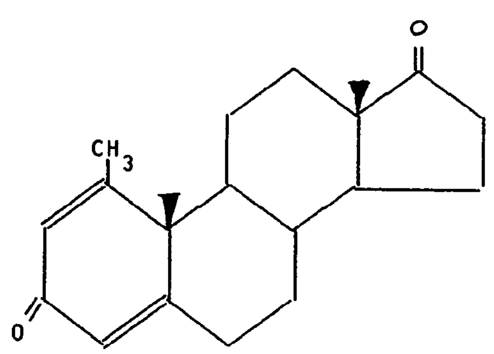

(I),

dadurch gekennzeichnet, daß man Androsta-1,4-dien-3,17-dion

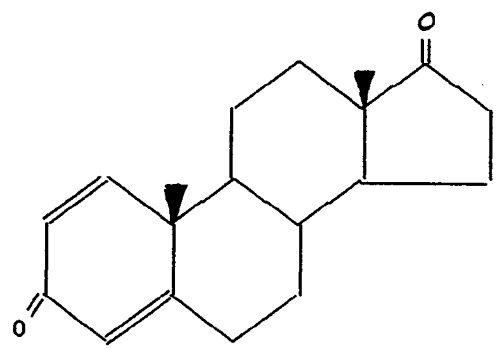

durch Umsetzung mit einem Methylanionen liefernden Reagenz und anschließendes Abfangen des gebildeten Enolats in einen 3-Enolester oder 3-Enolether der allgemeinen Formel II

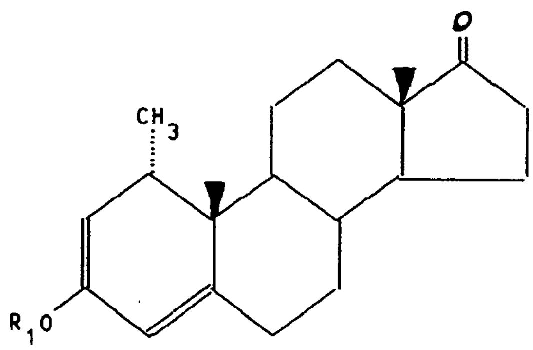

(II),

worin

$R_1$ einen Alkylrest mit 1-3 Kohlenstoffatomen oder eine Acyl- oder Trialkylsilylgruppe mit bis zu 10 Kohlenstoffatomen in der Gruppe bedeutet, überführt,

diesen mit elementarem Brom oder einem Bromierungsreagenz, bei dem das aktive Brom an amidischen Stickstoff gebunden vorliegt, in das Bromsteroid der Formel III

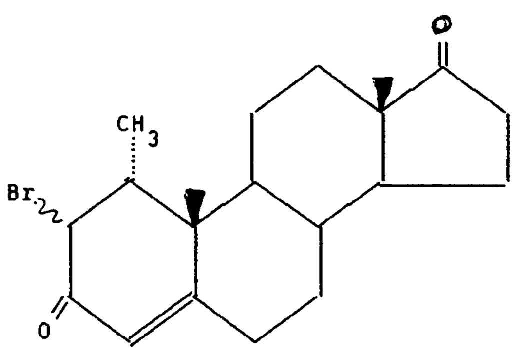

(III),

worin

der Bromsubstituent sowohl in der α- als auch in der β-Position stehen kann,

umsetzt, und die Verbindung der allgemeinen Formel III durch Dehydrobromierung in Gegenwart von Alkali- oder Erdalkylioxiden oder -carbonaten in die verbindung der allgemeinen Formel I überführt.

2. Verbindungen der allgemeinen Formel II

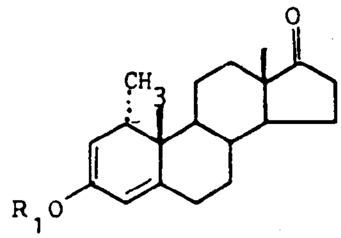

(II).

worin

$R_1$ einen Alkylrest mit 1-3 Kohlenstoffatomen oder eine Acyl- oder Trialkylsilylgruppe mit bis zu 10 Kohlenstoffatomen in der Gruppe bedeutet.

3. Verfahren gemäß Anspruch 1, dadurch gekennzeichnet, daß als Lösungsmittel für die Bromierung polare aprotische Lösungsmittel oder Wasser verwendet werden.

4. Verfahren gemäß Anspruch 3, dadurch gekennzeichnet, daß als Lösungsmittel Dioxan, Tetrahydrofuran, Aceton oder Dichlormethan oder Gemische aus diesen Lösungsmitteln verwendet werden.

5. Verfahren gemäß Anspruch 1, dadurch gekennzeichnet, daß als Lösungsmittel für die Dehydrobromierung Dimethylformamid, Dimethylacetamid, Dimethylimidazolidinon, Dimethylsulfoxid oder Wasser oder Gemische aus diesen Lösungsmitteln verwendet werden.

6. 3-Acetoxy-1α-methyl-androsta-2,4-dien-17-on.

7. 2α-Brom-1α-methyl-4-androsten-3,17-dion.

8. 2β-Brom-1α-methyl-4-androsten-3,17-dion.

## Claims

1. A process for the preparation of 1-methyl-androsta-1,4-diene-3,17-dione of formula I

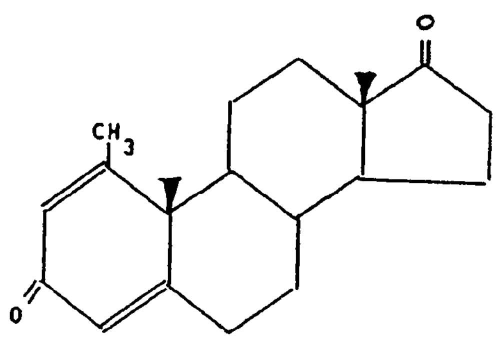

(I),

characterised in that androsta-1,4-diene-3,17-dione

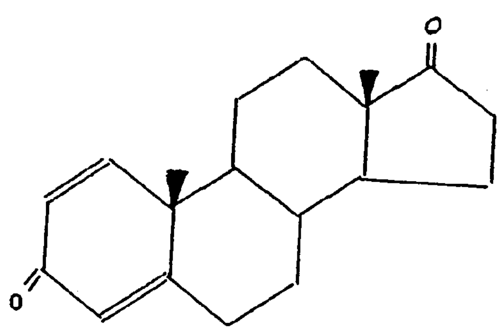

is converted, by reaction with a reagent that yields methyl anions and subsequent capture of the enolate that is formed, into a 3-enol ester or 3-enol ether of the general formula II

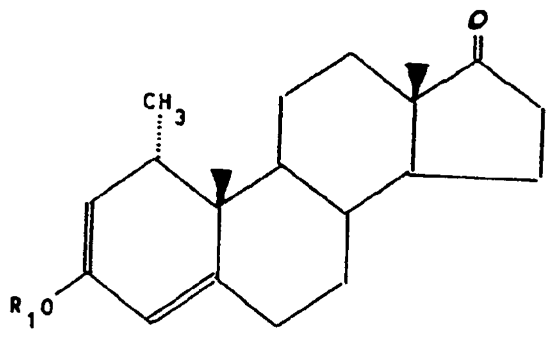

(II),

wherein

$R_1$ is an alkyl radical having from 1 to 3 carbon atoms or an acyl or trialkylsilyl group having up to 10 carbon atoms in the group,

the latter is reacted with elemental bromine or with a brominating reagent wherein the active bromine atom is present bound to amidic nitrogen to form the bromo steroid of formula III

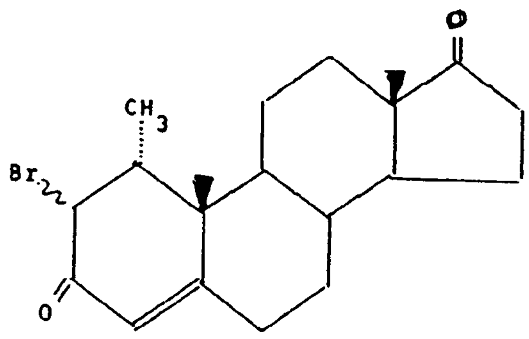

(III),

wherein

the bromine substituent may be in the α- as well as in the β-position,

and the compound of the general formula III is converted by dehydrobromination in the presence of alkali or alkaline earth metal oxides or carbonates into the compound of the general formula I.

2. Compounds of the general formula II

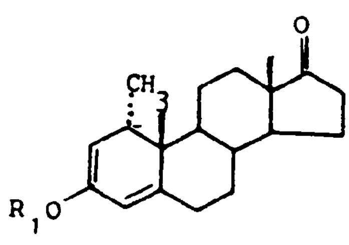

(II),

wherein

$R_1$ is an alkyl radical having from 1 to 3 carbon atoms or an acyl or trialkylsilyl group having up to 10 carbon atoms in the group.

3. A process according to claim 1, characterised in that polar aprotic solvents or water are used as solvents for the bromination.

4. A process according to claim 3, characterised in that dioxane, tetrahydrofuran, acetone or dichloromethane, or mixtures of those solvents, are used as the solvents.

5. A process according to claim 1, characterised in that dimethylformamide, dimethylacetamide, dimethylimidazolidinone, dimethyl sulfoxide or water, or mixtures of those solvents, are used as solvents for the dehydrobromination.

6. 3-Acetoxy-1α-methyl-androsta-2,4-dien-17-one.
7. 2α-Bromo-1α-methyl-4-androstene-3,17-dione.
8. 2β-Bromo-1α-methyl-4-androstene-3,17-dione.

## Revendications

1. Procédé pour préparer la méthyl-1 androstadiène-1,4 dione-3,17 de formule I :

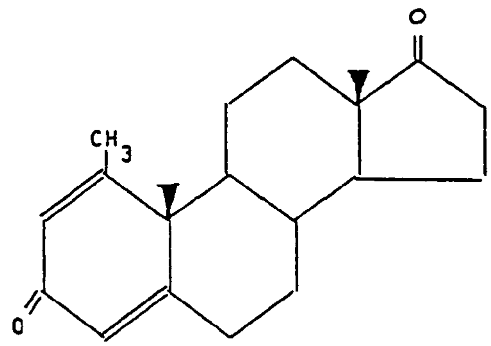

(I),

procédé caractérisé en ce qu'on transforme l'androstadiène-1,4 dione-3,17 :

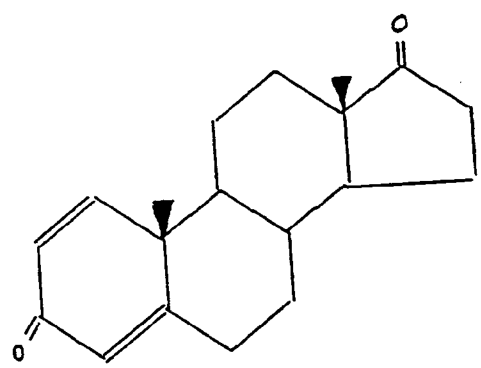

par réaction avec une substance fournissant des anions méthyles, puis fixation de l'énolate formé, en un ester ou éther d'énol en 3 répondant à la formule générale II :

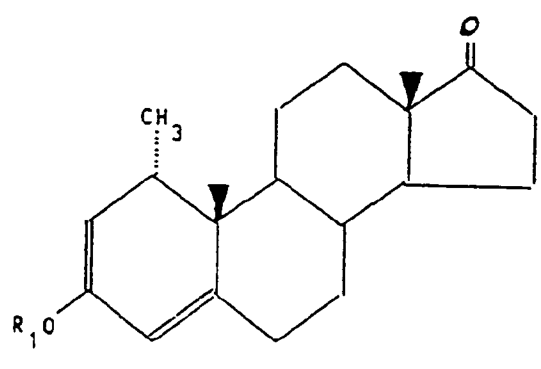

(II),

dans laquelle $R_1$ représente un radical alkyle contenant de 1 à 3 atomes de carbone ou un radical acyle ou trialkylsilyle contenant au maximum 10 atomes de carbone, on fait réagir celui-ci avec du brome élémentaire ou avec un réactif de bromation dans lequel le brome actif est lié à un azote amidique, de manière à obtenir le bromo stéroïde répondant à la formule III :

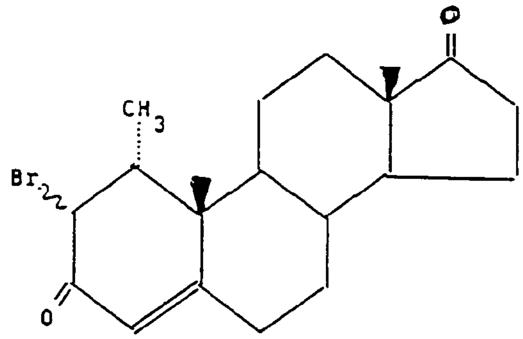

(III),

dans laquelle
le substituant brome peut avoir aussi bien la configuration α que la configuration β,
et on transforme le composé de formule générale III, par déshydrobromation en présence d'oxydes ou de carbonates de métaux alcalins ou de métaux alcalino-terreux, en le composé de formule générale I.

2. Composés répondant à la formule générale II

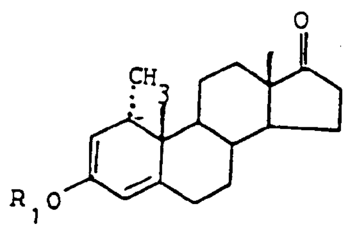

(II),

dans laquelle $R_1$ représente un radical alkyle contenant de 1 à 3 atomes de carbone ou un radical acyle ou trialkylsilyle contenant au plus 10 atomes de carbone dans le groupe.

3. Procédé selon la revendication 1 caractérisé en ce qu'on utilise, comme solvant pour la bromation, un solvant aprotique polaire ou de l'eau.

4. Procédé selon la revendication 3 caractérisé en ce qu'on utilise, comme solvants, du dioxanne, du tétrahydrofuranne, de l'acétone, du dichlorométhane ou des mélanges de ces solvants.

5. Procédé selon la revendication 1 caractérisé en ce qu'on utilise, comme solvants pour la déshydrobromation, du diméthylformamide, du diméthylacétamide, de la diméthylimidazolidinone, du diméthylsulfoxyde, de l'eau ou des mélanges de ces solvants.

6. Acétoxy-3 méthyl-1α androstadiène-2,4 one-17.

7. Bromo-2α méthyl-1α androstène-4 dione-3,17.

8. Bromo-2β méthyl-1α androstène-4 dione-3,17.